# EUROPEAN PATENT SPECIFICATION

(11) **EP 2 198 930 B1**
(45) Date of publication and mention of the grant of the patent: **09.11.2011**
(21) Application number: 10158833.3
(22) Date of filing: 22.08.2007
(51) Int. Cl.: A61Q 19/00, A61Q 19/10, A61K 8/39, A61K 8/44, A61K 8/81, A61K 8/73

(54) **Methods for incorporating a hydrophobic material into a skin care composition**
Verfahren zur Einarbeitung eines hydrophobischen Bestanteils in einer Hautpflegezusammensetzung
Méthode d'incorporation d'un matériau hydrophobe dans une composition de soin de la peau

(30) Priority: 23.08.2006 US 823305 P; 08.03.2007 US 683801
(43) Date of publication of application: 23.06.2010
(62) Divisional of application: 07814339.3
(73) Proprietor: Colgate-Palmolive Company, New York NY 10022-7499 (US)
(72) Inventor: Haskel, Ariel, East Brunswick, NJ 08816 (US); Pimenta, Paloma, Staten Island, NY 10309 (US)
(74) Representative: Jenkins, Peter David

(56) References cited:
- WO-A-2006/012372
- US-A1- 2005 158 268

## Description

This invention relates to a method for incorporating a hydrophobic material into a skin care composition.

### BACKGROUND OF THE INVENTION

Moisturizing formulations for skin care typically contain hydrophobic materials such as petrolatum or other oils or waxes which include liquids, solid or semi-solid at room temperature, in the form of oil-in-water emulsions. The hydrophobic materials are typically introduced into the aqueous medium by adding under shear stress a hot oil phase containing, *e.g.,* petrolatum, into a hot aqueous phase in the presence of emulsifiers to allow for homogenous dispersion of the petrolatum into the surfactant system. Simply incorporating melted petrolatum or similar material into a cold base is not feasible as the melted petrolatum solidifies upon contact with the base material. The need for hot processing, however, renders the manufacturing relatively expensive and difficult. Moreover, the emulsifiers can interfere with the desired deposition of the hydrophobic agents on the skin.

The applicant's earlier WO-A-2006/012372 discloses liquid cleaning compositions incorporating an acrylate polymer, a surfactant and shea butter beads.

### BRIEF SUMMARY OF THE INVENTION

The present invention provides a method of incorporating a hydrophobic material into a skin care composition, which comprises (a) forming a base comprising mixing an acrylate polymer and a surfactant; (b) forming a hydrophobic material premix comprising mixing a hydrophobic material and a yield value increasing cationic polymer; and (c) combining the hydrophobic material premix with the base, wherein the method is carried out at a temperature of no greater than 50°C.

Preferred features are defined in the dependent claims.

### BRIEF DESCRIPTION OF THE DRAWINGS

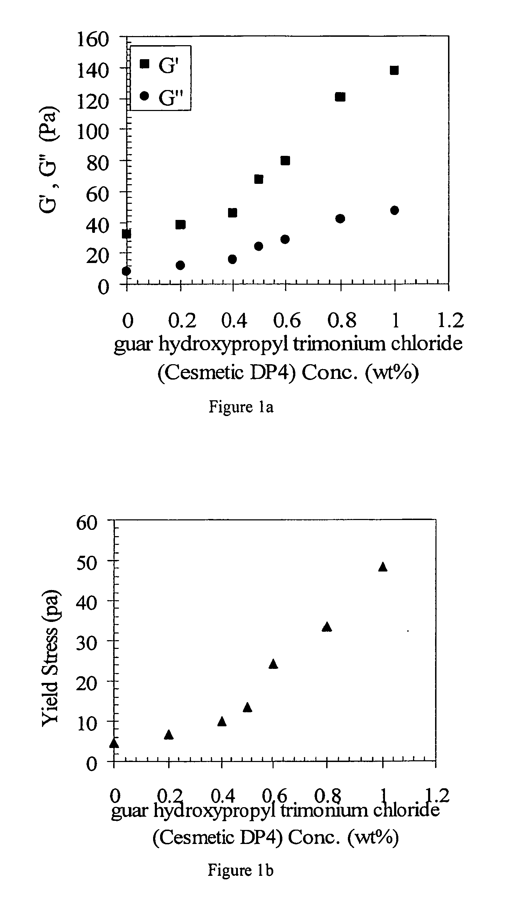

Figure 1 illustrates the effect of guar hydroxypropyl trimonium chloride (Cesmetic™ DP4) to the rheological parameters. Figure 1a illustrates the affect on G' and G". Figure 1b illustrates the effect on yield stress of the composition (35.28% sodium laureth sulfate with an average of 2 moles of ethylene oxide groups (SPES), 5.36%, cocamidopropyl betaine (CAPB), and 8.5% CARBOPOL™ Aqua SF1 polymer).

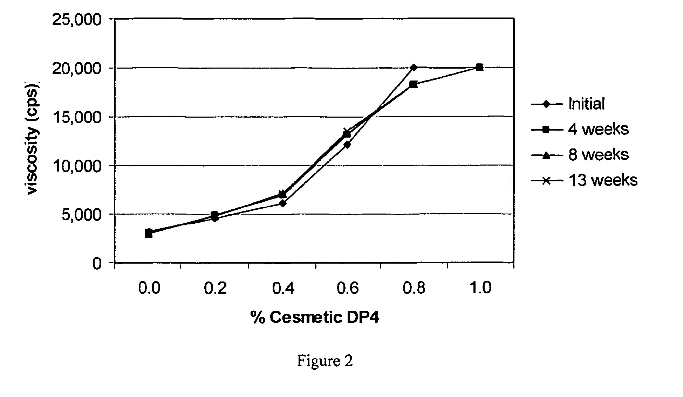

Figure 2 illustrates a viscosity profile of this composition (35.28% SPES, 5.36% CAPB, 8.5% CARBOPOL™ Aqua SF1 polymer) upon addition of guar hydroxypropyl trimonium chloride (Cesmetic™ DP4).

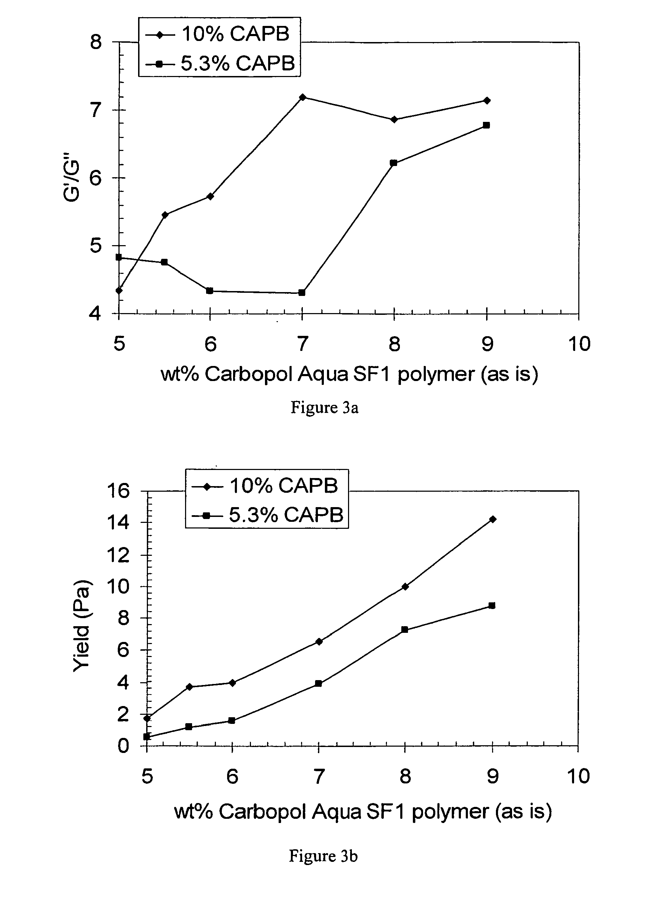

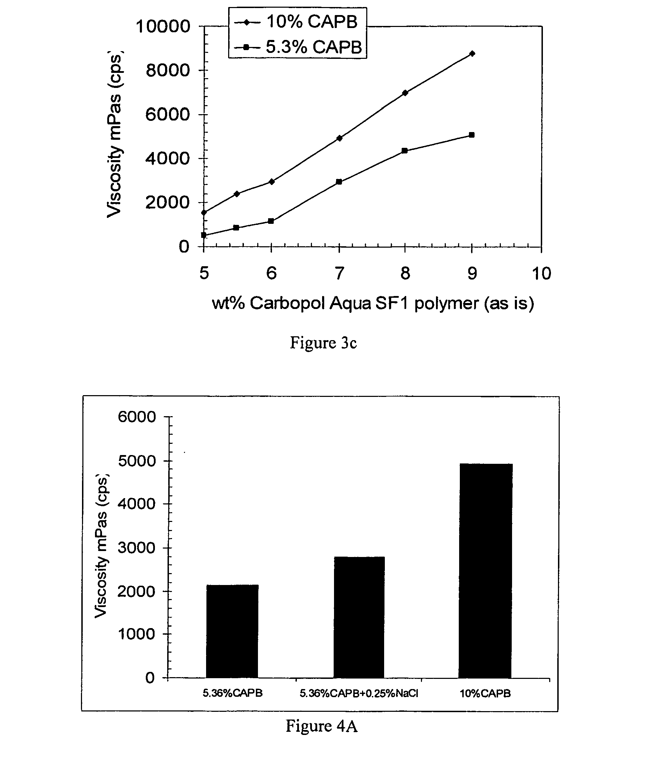

Figure 3 - (a) G'/G", (b) Yield Stress and (c) Viscosity; as a function of wt% of CARBOPOL™ Aqua SF1 acrylates copolymer for two different surfactant formulations: (1) 31.37 wt% SPES; 10.00 wt% CAPB and (2) 36.86% SPES; 5.36 wt % CAPB. Total active ingredients (AI) in both formulas is 11.0 wt% surfactants.

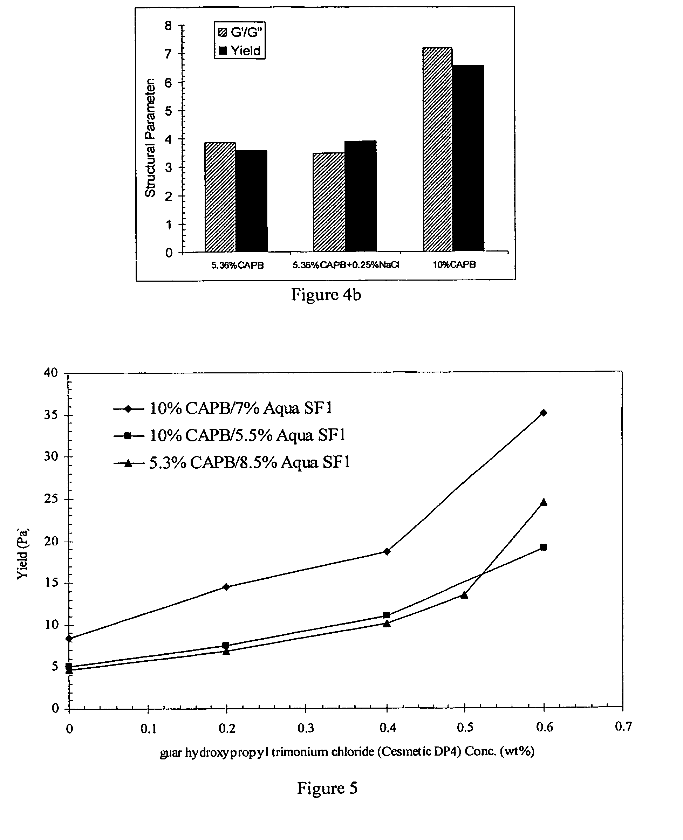

Figures 4a and b illustrate a comparison of viscosity (4a) and structural parameter values (4b) for the following formulations: a) 36.86% SPES, 5.36%CAPB, 7% CARBOPOL™ Aqua SF1 polymer; b) 36.86% SPES, 5.36% CAPB, 7% CARBOPOL™ Aqua Ski polymer, 0.25% NaCl; and c) 31.37% SPES, 10.0% CAPB, 7% CARBOPOL™ Aqua SF1 polymer.

Figure 5 illustrates the effect of Cesmetic™ DP4 guar hydroxypropyl trimonium chloride addition to the following compositions: (□) 35.28% SPES, 5.36% CAPB, 8.5% CARBOPOL™ Aqua SF1 polymer; (D) 29.8% SPES, 10% CAPB, 5.5% CARBOPOL™ Aqua SF1 polymer; (o) 29.8% SPES, 10% CAPB, 7% CARBOPOL™ Aqua SF1 polymer.

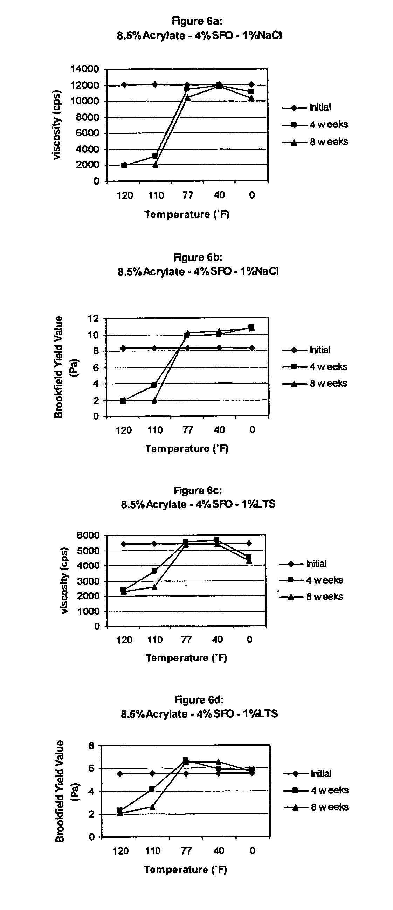

Figure 6 illustrates the viscosity of the composition containing either NaCl a) and b) or PROMIDIUM™ LTS blend of PEG-150 distearate and PPG-2 hydroxyethyl cocamide c) and d). Samples are aged at different temperatures and viscosities and measured after 4 and 8 weeks of aging. All viscosities are measured at room temperature.

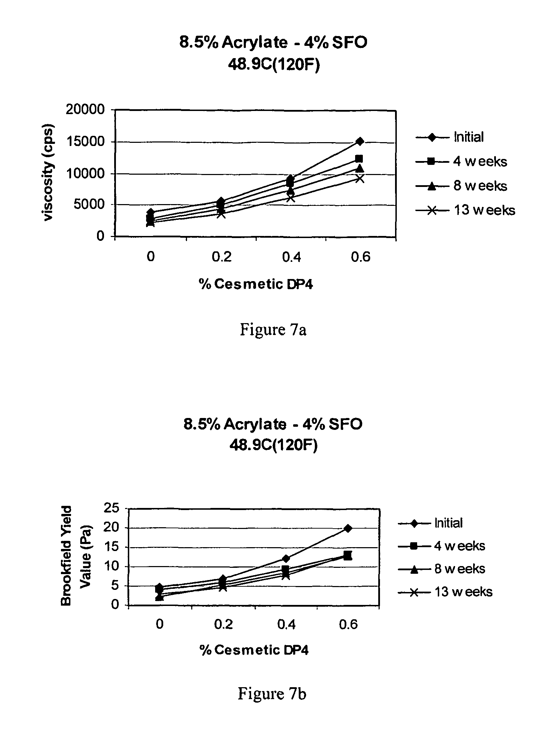

Figures 7a and 7b illustrate aging profiles of a composition containing surfactants/acrylates copolymer/guar quat and 4% sunflower oil (SFO) at 48.9°C (120°F) aging profile.

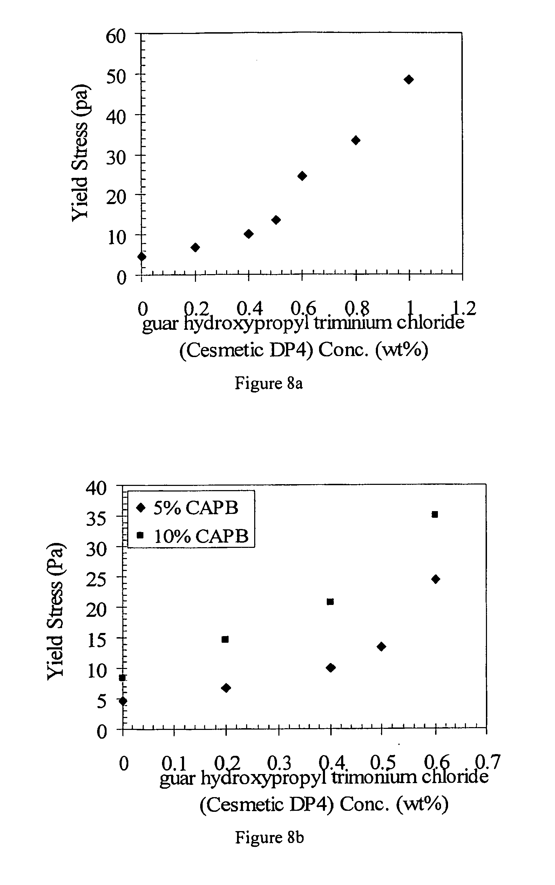

Figure 8a illustrates yield stress as a function of %wt Cesmetic™ DP4 guar hydroxypropyl trimonium chloride with 4% sunflower oil (SFO). Figure 8b compares the effect of Cesmetic™ DP4 guar hydroxypropyl trimonium chloride in the composition (a) versus another composition (29.8% SPES, 10% CAPB, and 7% CARBOPOL™ Aqua SF1 I polymer) with 4% sunflower oil (SFO) (□).

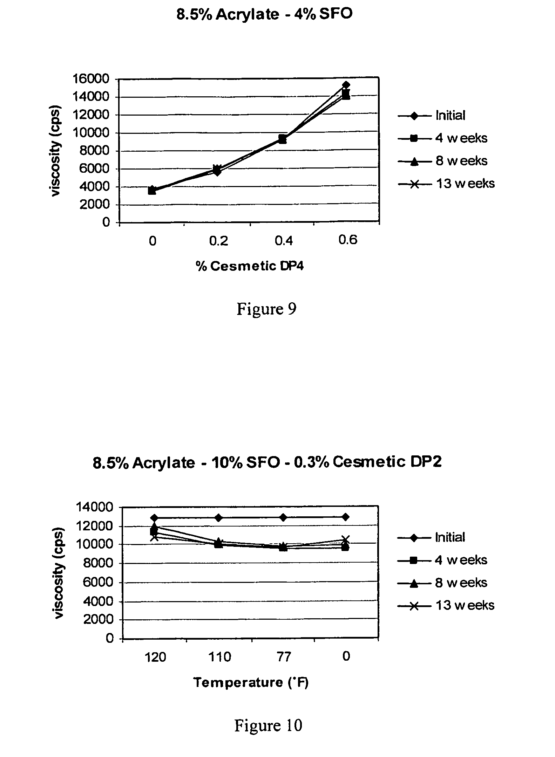

Figure 9 illustrates above composition aging profile at 25°C (77°F).

Figure 10 illustrates the viscosity of oil containing composition (with Cesmetic™ DP4 guar hydroxypropyl trimonium chloride) as a function of temperature.

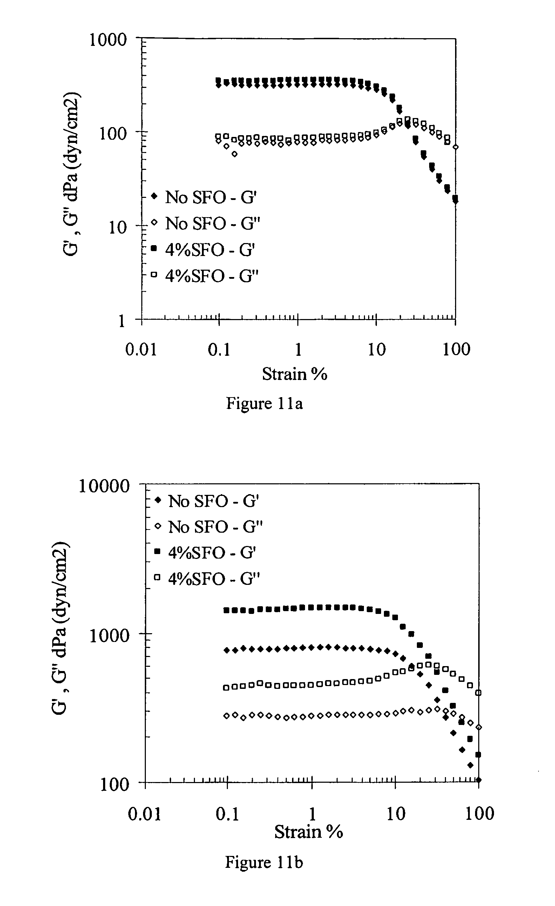

Figures 11a and 11b illustrate the effect on G' and G" dPa (dyne/cm²) versus strain for compositions with and without sunflower oil (SFO). Figure 11a is a composition without guar hydroxypropyl trimonium chloride (Cesmetic™ DP4), and Figure 11b is a composition with 0.6 weight% guar hydroxypropyl trimonium chloride (cosmetics* DP4).

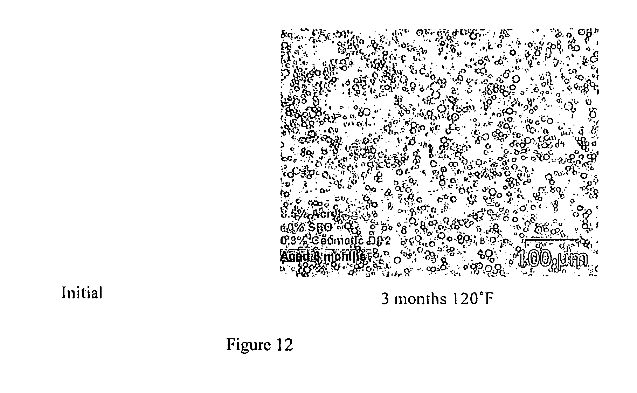

Figure 12 illustrates a micrograph showing stable suspended oil droplets in the described composition containing Cesmetic™ DP4 guar hydroxypropyl trimonium chloride after 3 months at 48.9°C (120°F).

### DETAILED DESCRIPTION OF THE INVENTION

As used throughout, ranges are used as a shorthand for describing each and every value that is within the range. Any value within the range can be selected as the terminus of the range. All references cited herein are incorporated herein by reference. If there is any conflict in a definition of a term between a reference and this specification, the definition in this specification shall control.

As used throughout, amounts of materials are based on supplied amounts, which includes the active amount of material and the amount of any carrier for the material.

Described herein are skin care compositions that may be prepared by cold processes and include at least an acrylate polymer, a yield value increaser, and a surfactant. In one embodiment, the composition has a yield value of at least about 4.

The skin care composition may be a substantially non-emulsified composition. The term "substantially non-emulsified" as used herein means a composition other than a conventional emulsion, or a composition which is not an O/W type emulsion or a W/O type emulsion. In other words, when two solutions are mixed one phase is not dispersed in the other phase in the form of micelles that are completely stabilized by an emulsifying agent.

By "cold process" it is meant that at least the step of incorporating the hydrophobic material into the final composition may be carried out at ambient temperature, for example, about 10°C to about 50°C, or about 15°C to about 30°C, up to about 45°C, up to about 40°C, up to about 35°C, or up to about 30°C.

It has surprisingly been discovered that the structure of formulations containing an acrylate copolymer can be kept the same or increased by (1) using an amount of a salt of guar hydroxypropyl trimonium or (2) using a modified surfactant system by selecting the ratio of zwitterionic surfactant to anionic surfactant, for example, higher cocamidopropyl betaine (CAPB) and lower sodium laureth sulfate with an average of 2 moles of ethylene oxide groups (SPES) in order to lower the amount of CARBOPOL™ Aqua SF1 polymer necessary to obtain a structured system. Thus, the inventors have found a method of enhancing the performance of the CARBOPOL™ Aqua SF1 polymer by modifying the zwitterionic surfactant to anionic surfactant ratio (CAPB:SPES ratio) in the formulation.

The composition includes an acrylate homopolymer or copolymer (hereinafter collectively referred to as "polymer"). The acrylate polymer may be any known or to be developed in the art and may include swellable acrylate co-polymer, *e.g.,* such as CARBOPOL™ Aqua SF-1 (from Noveon, Cleveland, Ohio, United States of America). Suitable polymers (including homopolymers or co-polymers of the listed components) may include those of 2-hydroxyethyl acrylate, hydroxypropyl acrylate, polymers of acrylic acid and its esters, polymers of methacrylic acid and its esters, acrylnitriles, ethylacrylate, methacrylate, polyalkenyl ethers of sucrose, polyalkenyl ethers of polyalcohols, trimethylpropone tri(meth)acrylate, glycidal methacrylate, and N-methylolacryamide. Other polymers that may be suitable are described in United States Patent No. 6,635,702, the contents of which are incorporated herein by reference. Mixtures of polymers (co- and homo-) may also be used, if desired.

The selected polymer(s) may be present in the composition in any amount. In certain embodiments, they are present in an amount of about 1% to about 30% by weight of the total skin care composition, with amounts of about 3% to about 12%, and about 5% to 12% of the total weight of the skin care composition also being suitable.

The composition produced in accordance with the invention includes a yield-increasing polymer that is different from the acrylate polymer. By "yield value increaser" it is meant a component, for example, a salt of guar hydroxypropyl trimonium or a modified surfactant system, when added to an acrylate polymer, the yield value increased by about at least 5%, about 10%, about 15%, about 20%, about 25%, about 30%, about 40% or more (yield values measured as described herein).

The yield value increaser is a cationic polymer. It may be, for example, a cationic gum. The cationic polymer may be a modified guar gum or other gum, such as a salt of guar hydroxypropyl trimonium, hydroxypropyl guar and/or C₁₈ to C₁₂ alkyl hydroxypropyl guar. The salt of guar hydroxypropyl trimonium may be, *e.g.,* any containing a suitable anion, such as guar hydroxypropyl trimonium chloride.

The cationic polymer may be present in the composition in any amount. In one embodiment, it is present in an amount of up to about 20% by weight. Alternatively, amounts may be up to about 10% by weight, about 0.1 % to about 5% by weight and about 7% to about 12% by weight.

The skin care composition includes a hydrophobic material, which may be a non-silicone hydrophobic material. Such material may be liquid, solid or semi-solid at room temperature and may function to confer a skin benefit when applied topically, *e.g.,* skin moisturization. Exemplary hydrophobic materials include: mineral oils, synthetic oils, vegetable oils, semi-solid hydrocarbons, petrolatum, long chain alkanes and alkenes, isodedecane, isohexadecane, hydrogenated polydecene, polydecene, and hydrocarbons found in beeswax (for example, C₂₁₋₃₇ hydrocarbons), esters of fatty acids (including hydrogenated forms or derivatives thereof) and long straight chain alcohols, triacontanol hexadecanoate, hexacosanol hexacosanoate, and myricyl palmitate, fatty acid triglycerides, animal fats, lanolin, hydrogenated or partially hydrogenated vegetable oils, hydrogenated or partially hydrogenated sunflower oil, safflower oil, soybean oil, rapeseed oil, grape seed oil, corn oil, olive oil, sweet almond oil, coconut oil, palm kernel oil, soybean oil, and/or derivatives thereof, castoryl maleate, hydrophobic vegetable extracts, shea butter, cocoa butter, and derivatives and fractions thereof; hydrophobic UV absorbers, for example cinnamates, octinoxate, benzophenes and benzophenone, oxybenzone, salicylates, octisalate, anthranilates, and p-anunobenzoic acid esters, water-insoluble vitamins, including water-insoluble vitamin derivatives, vitamin A, vitamin D2, vitamin D3, vitamin E, vitamin E acetate, and water-insoluble pharmaceuticals for topical application, antibiotic agents, antifungal agents, antibacterial agents, analgesic agents, anti-inflammatory agents, an organic sunscreen, an agent coated with organic material, a synthetic oil, a hydrophobically coated particle, a liquid organic material containing a hydrophobic material, an insect repellent, a semi-solid hydrocarbon. The hydrophobic material can be present as a homogenous dispersion.

The hydrophobic material may be present in any amount. In one embodiment, it is present in an amount of about 0.2% to about 20% by weight of the total skin care composition or about 4% to about 10% by total weight of the skin care composition.

The hydrophobic material may be present in the skin care composition in a non-conventional-emulsion form; it may be present or substantially present as a suspended non-emulsified droplet or dispersion. Accordingly, the skin care composition itself may be referred to as a substantially emulsifier free composition. In certain embodiments, at least about 80%, about 85%, about 90%, about 95%, and about 98% of the hydrophobic material is present as non-emulsified droplets or dispersions. The droplets or dispersions may be homogenous or they may be mixtures or emulsifications of other hydrophobic or hydrophilic materials.

In certain embodiments, the droplets/dispersions have an average diameter of about 0.01 to about 100 microns, about 0.1 to about 10 microns, or about 1 micron to about 50 microns.

The composition has a yield value of at least about 4. In some embodiments, the compositions may have a yield value of about 5 to about 10 or about 4 to about 25. Yield values are measured using a Brookfield Yield Rheometer YR-1 at ambient temperature (25°C), using the spindle # 72.

The composition includes a surfactant or mixture of surfactants. The base composition contains a surfactant. As mentioned above, one embodiment of the invention provides a selected ratio of zwitterionic surfactant to anionic surfactant. However, any surfactant suitable for a skin cleansing product may be incorporated. In addition to the selected ratio of zwitterionic surfactant to anionic surfactant, the compositions may also optionally include one or more additional surfactants.

Suitable surfactants may include a surfactant or mixture of surfactants having a combined HLB value which is at least about one HLB unit higher than the HLB required for desired emulsion of the hydrophobic material used in the skin care composition. In certain embodiments, the HLB is at least about two HLB units higher than the HLB required for desired emulsion. The HLB of the surfactant/surfactant may be, for example, at least about 10, at least about 12, or at least about 14.

Surfactants may include amphoteric, sulfate, zwitterionic, sulfonate, anionic surfactants, such as, for example, lauryl sulfates, lauryl ether sulfates, sodium lauryl sulfate, sodium laureth sulfate, sodium methyl-2 sulfolaurate, disodium 2-sulfolaurate, sodium lauryl sulfoacetate, disodium laureth sulfosuccinate, amphoteric acetates, sodium laurylamphoacetate, betaine derivatives, cocoamidopropyl betaine (CAP-Betaine), alkyl amines, alkyl imidazolines, cetylpyridinium chloride, PEG-50 stearamine, and dimethyl palmitamine.

One or more surfactants may be included in the compositions. In one embodiment, the selected surfactants are sulfate surfactant(s) and amphoteric surfactant(s), which can in one embodiment be present in a weight ratio of about 2:1 to about 4:1 or about 3:1.

The surfactant(s) may be present in the skin care composition in any amount. For example, amounts of about 4% to 20% by weight of the total composition or about 12% to about 18% of the total composition may be present. Alternatively, water may be present in amounts of at least about 90% by weight or at least about 95% by weight of the total composition.

It has been surprisingly found that the addition of small quantities of a viscosity modifier, for example, a salt of guar hydroxypropyl trimonium (e.g., guar hydroxypropyl trimonium chloride (Cesmetic™ DP4/DP2. Rita/Lamberti)), into a surfactant base significantly improves the structural properties of this formulation. Both G' (the elastic modulus) and G" (the viscous modulus) significantly increase upon addition of Cesmetic™ DP2 or DP4 guar hydroxypropyl trimonium chloride as illustrated in Figure 1a. The yield stress value illustrated in Figure 1b, which is a good measure of the suspending capabilities of the system, also increased significantly. In one embodiment, it has been found that the effect is maximized for DP4 concentrations greater than 0.4 wt%.

In certain illustrative embodiments, the Brookfield viscosity also improved significantly upon adding guar hydroxypropyl trimonium chloride to the structured system so that targeted product viscosity can be achieved without the further addition of salt as illustrated in Figure 2. This becomes especially useful in formulations in which salt may jeopardize product stability. In another illustrative embodiment, the effect of guar hydroxypropyl trimonium chloride (*i.e.,* Cesmetic™ DP4) on formulations with different surfactant to co-surfactant ratios was compared.

In addition, it has been surprisingly found that the structural properties of the surfactant base, for example, CARBOPOL™ Aqua SF1 polymer containing bases, can be greatly improved by increasing the zwitterionic surfactant to anionic surfactant ratio (e.g., ratio of CAP-Betaine to SPES) in the formulation, while maintaining the total surfactant concentration constant.

Rheology data in Figure 3 illustrate the effect on illustrative embodiments of CARBOPOL™ Aqua SF1 polymer on G'/G" (ratio of elastic to viscous moduli; Figure 3a), on the yield stress value (Figure 3 b), and on the viscosity (Figure 3c), for two formulas with different CAP-Betaine to SPES ratios at 11 % total active ingredient (AI) for surfactants. In certain illustrative embodiments, it was found that increasing the amount of CAP-Betaine from 5.36 wt% (as is) to 10.0 wt% (as is) significantly improves the performance of the acrylates copolymer; i.e. higher G'/G", yield and viscosity values are achieved using less of the structuring agent CARBOPOL™ Aqua SF1 polymer. For example, in an illustrative embodiment at 6 wt%, CARBOPOL™ Aqua SF1 polymer, the change in surfactant ratio leads to a 32% increase in G'/G", a 151 % increase in the yield value, and a 158% increase in viscosity.

Figure 4 compares an illustrative formulation containing 31.37% SPES, 5.36% CAPB and 7% CARBOPOL™ Aqua SF1 polymer to the same system with 0.25% NaCI. As illustrated, the amount of NaCl that CAP-Betaine contributes to the formulation slightly increases the viscosity of the system but has no significant effect on the structure parameters, i.e. yield value and G'/G" ratio. These results illustrate that the structure enhancement observed from the increase in the CAPB to SPES ratio is in fact due to the change in surfactant to co-surfactant ratio.

Rheology data in Figure 5 illustrate the yield point values for three different bases as a function of added guar hydroxypropyl trimonium chloride (Cesmestic™ DP4). In each case, addition of guar hydroxypropyl trimonium chloride increased the yield point value. In certain embodiments, the effect is enhanced by the increase in surfactant to co-surfactant ratio. The composition containing 10% CAPB and 5.5% CARBOPOL™ Aqua SF1 polymer showed equal performance when compared to another composition (5.36% CAPB, 8.5% CARBOPOL™ Aqua SF1 polymer). On the other hand, in another illustrative embodiment, a base containing 10% CAPB and 7% CARBOPOL™ Aqua SF1 polymer displayed greater yield values than the two previously mentioned bases. It has been surprisingly found that the addition of guar hydroxypropyl trimonium chloride (Cesmetic™ DP2 and Cesmetic™ DP4) provides better product stability in suspending oils in liquids. In one embodiment, the addition of guar hydroxypropyl trimonium chloride during certain cold processable technologies, for example, current manufacturing processes that simply allow for post-addition of the hydrophobic materials to the composition, the addition of guar hydroxypropyl trimonium chloride increased the stability of oils in liquid.

Figure 6 depicts the effect with NaCl (Figure 6a-6b) and PROMIDIUM™ LTS blend of PEG-150 distearate and PPG-2 hydroxyethyl cocamide (Figure 6c-6d) where viscosities and Brookfield values remain fairly stable below 25°C (77°F) but decrease at higher temperatures. In certain embodiments, addition of 0.2% to 1% DP4 to 95% of a composition (35.28%SPES, 5.36%CAP-Betaine, 8.5%CARBOPOL™ Aqua SF1 polymer) in the presence of 4% sunflower oil (SFO) produces an increase in viscosity (Figure 7 a) and yield stress (Figure 7b). The latter indicating improved structure and suspension capabilities. In certain embodiments, the increase in yield stress is dependent upon surfactant to co-surfactant ratio and amount of acrylate co-polymer present as illustrated in Figures 8a and 8b. In certain embodiments, higher levels of CAP-Betaine boost the suspension capabilities of the system at lower SPES and CARBOPOL™ Aqua SF1 polymer levels than the original composition.

As illustrated in Figure 9, the high temperature viscosities and yield values aging profiles show fluctuations, which are small compared to the NaCl (Figure 6a and 6b) and PROMIDIUM™ LTS blend of PEG-150 distearate and PPG-2 hydroxyethyl cocamide (Figure 6c and 6d) profiles. In a certain embodiment, this technology allows for the stabilization of at least 10% sunflower oil as shown in the viscosity aging profile (Figure 10) and oil droplet suspension micrographs (Figure 12).

The addition of guar hydroxypropyl trimonium chloride to a cleanser containing an acrylate polymer, and sunflower oil provided an unexpected increase in yield value. While the viscosity was expected to increase, the synergistic effect of the guar with the polymer increased the yield value of the composition. Additionally, the addition of sunflower oil provides a further increase in yield when combined with the acrylate polymer and the guar.

In order to understand the mechanism of action of this guar material, the rheology of the composition with and without sunflower oil and guar hydroxypropyl trimonium chloride (Cesmetic™ DP4) was compared. Figures 11 illustrate the behavior of the elastic and loss moduli, G' and G" respectively, as a function of % strain. In a certain embodiment, the addition of the guar to the composition increases both G' and G" (see open symbols in Figure 11a and 11b). In another embodiment, the addition of sunflower oil to the composition in the absence of the guar has no effect on the rheological parameters (Figure 11a). In another embodiment, if the structured formula contains both DP4 and sunflower oil a further increase in both G' and G" takes place compared to non-oil containing formulas (Figure 11b).

Without being limited by theory, the surprising and unexpected benefits provided by addition of guar hydroxypropyl trimonium chloride to the composition in the presence of sunflower oil illustrated the existence of a synergistic effect between the components of the structured system, guar hydroxypropyl trimonium chloride and sunflower oil.

Water may also be included in the composition. It may be present in an amount, for example, of at least 40% by weight of the total composition. Alternatively, the composition may contain water in an amount of at least about 10%, at least about 20%, at least about 30%, at least about 50%, at least about 60%, or at least about 70%, each by weight of the total composition.

The formulation may optionally further include one or more viscosity modifiers in addition to the acrylate co-polymer, for example a cationic polymer. Any may be used - examples include chitosan, vegetable or marine colloids, and starches. If included, the skin care composition may contain, in one embodiment, about 0.1 % to about 2% by weight of the cationic polymer, based on the total weight of the skin care composition. In other embodiments, the amount can be at least about 0.4% or about 0.4% to about 1.5%.

The skin care compositions described herein may also contain any additional additives as are desired. Such additives may include insoluble particles, such as beads, pigments, polyethylene beads, natural particulates, encapsulates, shea butter in gelatin encapsulate, preservatives, chelators, ethylenediaminetetraacetic acid (EDTA), antibacterial agents, 1,3-dimethylol-5,5-dimethyl hydantoin (DMDMH), triclosan, or trichlocarbon, antioxidant agents, tocopheryl acetate, perfume, coloring agents, organic sunscreen agents, physical sunscreens, vitamins, creatine or retinoic acid. Other additives may include antimycotic agents, anti-inflammatory agents, menthol, adducts of an oil.

In some embodiment, the skin care compositions described herein may have a viscosity of about 2500 to about 25,000 cps, or about 3500 to about 15,000 cps, as measured using a Brookfield DV-II+ viscometer, with settings at spindle: 5, speed: 20 rpm, time: 60 sec.

It has been found that the skin care compositions described herein provide useful means for delivering dermatologically beneficial compounds to the skin. For example, the skin care composition can be formulated to contain a topically active or protective compound, *e.g.,* a sunscreen compound or vitamin, even when the active or protective compound is highly hydrophobic. The composition may be used to deliver the hydrophobic material and/or an additional, active compound to the skin surface. Specifically, one aspect of the invention includes a method of depositing a hydrophobic material on the epidermal surface. Such method includes applying to an epidermal surface any one of the compositions described herein, and subsequently removing the composition from the epidermal surface, such that at least a portion of the hydrophobic material and/or any additional, active compound(s), remain deposited on the epidermal surface. Removal of the composition can be accomplished by any means, including rinsing (with water or other fluid), wiping, blotting, scraping, evaporating and/or brushing.

The skin care compositions produced according to the method of the invention may be prepared by any process or procedures known or to be developed in the art. An exemplary process may include pre-mixing (*e.g.,* by shear mixing or other means of incorporation) the selected hydrophobic material(s) and the cationic polymer and a surfactant/acrylate copolymer in an aqueous solution to obtain a homogeneous suspension of droplets of hydrophobic material. In one embodiment, a premix of the hydrophobic material, and the cationic polymer is dispersed in the premix. The premix is then added to the surfactant base containing the acrylate polymer. If desired, the pH may be adjusted to obtain the desired viscosity or other properties of the formulation. At least this mixing step may be carried out at cold process temperatures described above. Any other additives may be mixed in subsequently. The method may be adapted or expanded to include preparation of any of the compositions described herein and may further be carried out as a cold process, as defined herein.

### EXAMPLES

### Example 1:

A skin care composition that was a body wash is prepared by mixing the first seven ingredients of Table I, below, to form a base (see United States Patent No. 6,635,702). To that base is added a premix composed of hydrogenated polydecene, octinoxate and guar hydroxypropyl trimonium chloride and the remaining ingredients. The resulting composition was substantially emulsifier-free.

**Table I**

| **Ingredient** | **Percentage (wt%)** |
|---|---|
| Water | q.s. |
| CARBOPOL™ Aqua SF-1 (30% solution) | 6.0 |
| Sodium Laureth Sulfate (25.5% solution) | 34 |
| Sodium Hydroxide (50% solution) | 0.7 |
| Cocoamidopropyl betaine (30% solution) | 6.0 |
| DMDMH (54% solution) | 0.4 |
| EDTA (40% solution) | 2.3920 |
| Hydrogenated Polydecene (25% solution) | 4.0 |
| Guar hydroxypropyl trimonium chloride | 0.5 |
| Octinoxate | 4.0 |
| StepanMild™ PCL (sodium methyl-2 sulfolaurate and disodium 2-sulfolaurate, sodium lauryl sulfoacetate) | 5.0 |
| Citric acid (50% solution) | 0.5 |

### Example 2:

A body wash composition containing Vitamin E Acetate is prepared as described above. In this example, however, the premix containing the active hydrophobic material (i.e Vitamin E Acetate) is either composed of sunflower oil and petrolatum or hydrogenated polydecene (Table III). These two compositions were compared against the control (Table II) on deposition performance.

**Table II**

| | |
|---|---|
| Demineralized Water | 48.70771 |
| DMDM Hydantoin | 0.41069 |
| Polyquaternium-7 | 2.568 |
| Sodium Laureth Sulfate (25.5%) | 33.0489 |
| Cocoamidopropyl betaine (30%) | 10.2764 |
| Decyl Glucoside (50%) | 2.3122 |
| EDTA (39%) | 0.2055 |
| Glycerin | 0.2 |
| Euperlan™ PK300 pearlescent concentrate | 1.0 |
| Fragrance | 1.17 |
| Tocopheryl Acetate | 0.10006 |

**Table III**

| Test Compositions | Mean µg Vitamin E Acetate/cm² skin | |
|---|---|---|
| | Post Wash - 1 | Post Wash - 4 |
| 0.1% Vitamin E Acetate + 4% Sunflower Oil + 2% Petrolatum | 0.406 | 0.728 |
| 0.1% Vitamin E Acetate + 4% Puresyn 6 | 0.258 | 0.228 |
| CONTROL +0.1% Vitamin E Acetate | 0.026 | 0.102 |

### Example 3:

A base composition containing a base structure of 35 wt% of sodium laureth sulfate, 5 wt% of cocoamidopropyl betaine, 8.5 wt% of an acrylate copolymer (CARBOPOL™ Aqua SF-1), and water (remaining content) is produced in accordance with Example 1. With the base composition, test compositions containing guar hydroxypropyl trimonium chloride (CESMETIC™ DP4), in the wt% concentrations of 0.2, 0.4, 0.6, 0.8 and 1.0% are produced. Viscosity is measured using Brookfield DV-II spindle #5, 20 rpm and yield value measured using a Brookfield Yield Rheometer YR-1, spindle 72.

**Table IV**

| % Guar hydroxypropyl trimonium chloride by weight | Viscosity (cps) | Yield value (Pa) |
|---|---|---|
| 0.0 | 3220 | 4.02 |
| 0.2 | 4540 | 5.45 |
| 0.4 | 6160 | 7.38 |
| 0.8 | 12120 | 17.04 |
| 0.8 | > 20000 | > 20 |
| 1.0 | > 20000 | > 20 |

The results demonstrate that the addition of small quantities of a yield-increasing polymer, *e.g.,* guar hydroxypropyl trimonium chloride, into the acrylate copolymer containing composition significantly improves structural properties of this formulation. In addition, the elastic modulus and the viscous modulus increase upon addition of the cationic polymer, as well as the yield stress value, which is a good measure of the suspending capabilities of the system. The effect is maximized when the concentration of the cationic polymer is greater than 0.4 wt%. The Brookfield viscosity improves upon adding the yield-increasing polymer to the system so that targeted product viscosity can be achieved without the further addition of salt.

## Claims

1. A method for incorporating a hydrophobic material into a skin care composition comprising:
a. forming a base comprising mixing an acrylate polymer and a surfactant;
b. forming a hydrophobic material premix comprising mixing a hydrophobic material and a yield value increasing cationic polymer; and
c. combining the hydrophobic material premix with the base,
wherein the method is carried out at a temperature of no greater than 50°C.

2. The method of claim 1, wherein the yield value increasing polymer is a modified guar gum.

3. The method of claim 1, wherein the yield value increaser is guar hydroxypropyl trimonium chloride.

4. The method of claim 1, wherein the acrylate polymer is at least one material chosen from a polymer of acrylic acid, a polymer of methacrylic acid, ethylacrylate, methylacryalate, polyalkenyl ethers of sucrose or polyalchols, trimethylolpropane tri(meth)acrylate, glycidyl methacrylate, and N-methylolacryamide.

5. The method of claim 1, wherein the hydrophobic material is at least one material chosen from an oil, hydrogenated polydecene, a vitamin, a vegetable oil, a mineral oil, an organic sunscreen, sunflower oil, petrolatum, an agent coated with organic material, a synthetic oil, a hydrophobically coated particle, a liquid organic material containing a hydrophobic material, and an insect repellent.

## Patentansprüche

1. Verfahren zur Einbringung hydrophoben Materials in eine Hautpflegezusammensetzung, bei dem:
a. eine Basis durch Mischen eines Acrylatpolymers und eines Tensids gebildet wird,
b. eine Vormischung mit hydrophoben Material durch Mischen eines hydrophoben Materials mit einem kationischem Polymer gebildet wird, das die Fließgrenze erhöht,
c. die Vormischung mit hydrophoben Material mit der Basis gemischt wird,
wobei das Verfahren bei einer Temperatur von nicht mehr als 50°C durchgeführt wird.

2. Verfahren nach Anspruch 1, bei dem das die Fließgrenze erhöhende Polymer ein modifiziertes Guargummi ist.

3. Verfahren nach Anspruch 1, bei dem der Fließgrenzenerhöher Guarhydroxypropyltrimoniumchlorid ist

4. Verfahren nach Anspruch 1, bei dem das Acrylatpolymer mindestens ein Material ist, das aus einem Acrylsäurepolymer, einem Methacrylsäurepolymer, Ethylacrylat, Methylacrylat, Polyalkenylethern von Saccharose oder Polyalkoholen, Trimethylolpropan, Tri(meth)acrylat, Glycidylmethacrylat und N-Methylolacrylamide ausgewählt ist.

5. Verfahren nach Anspruch 1, bei dem das hydrophobe Material mindestens ein Material ist, das aus einem Öl, hydriertem Polydecen, einem Vitamin, einem Pflanzenöl, einem Mineralöl, einem organischen Sonnenschutzmittel, Sonnenblumenöl, Petrolatum, einem mit einem organischem Material beschichteten Mittel, einem synthetischen Öl, hydrophob beschichteten Teilchen, einem flüssigen organischen Material, das ein hydrophobes Material enthält und einem Insektenschutzmittel ausgewählt ist.

## Revendications

1. Procédé pour incorporer un matériau hydrophobe dans une composition de soin de la peau consistant à :
a) former une base comprenant le mélange d'un polymère d'acrylate et d'un tensioactif ;
b) former un pré-mélange d'un matériau hydrophobe comprenant le mélange d'un matériau hydrophobe et d'un polymère cationique augmentant l'indice de fluidité ; et
c) combiner le pré-mélange d'un matériau hydrophobe avec la base,
lequel procédé est réalisé à une température non supérieure à 50°C.

2. Procédé selon la revendication 1, dans lequel le polymère augmentant l'indice de fluidité est une gomme de guar modifiée.

3. Procédé selon la revendication 1, dans lequel l'agent augmentant l'indice de fluidité est un chlorure de guar hydroxypropyl trimonium.

4. Procédé selon la revendication 1, dans lequel le polymère d'acrylate est au moins un matériau choisi parmi un polymère d'acide acrylique, un polymère d'acide méthacrylique, un éthylacrylate, un méthylacrylate, des polyalcényle éthers de sucrose ou de polyalcools, un triméthylolopropane tri(méth)acrylate, un glycidyl méthacrylate, et un N-méthylolacrylamide.

5. Procédé selon la revendication 1, dans lequel le matériau hydrophobe est au moins un matériau choisi parmi une huile, un polydécène hydrogéné, une vitamine, une huile végétale, une huile minérale, un écran solaire organique, une huile de tournesol, du pétrolatum, un agent revêtu d'un matériau organique, une huile synthétique, une particule revêtue de manière hydrophobe, un matériau organique liquide contenant un matériau hydrophobe, et un répulsif à insectes.
